# EUROPÄISCHE PATENTSCHRIFT

(11) **EP 0 368 292 B1**
(45) Veröffentlichungstag und Bekanntmachung des Hinweises auf die Patenterteilung: **26.01.1994**
(21) Anmeldenummer: 89120725.0
(22) Anmeldetag: 08.11.1989
(51) Int. Cl.: C07C 407/00, C07C 409/08, C07C 409/10, C07C 409/12

(54) **Verfahren zur Herstellung von Aralkylhydroperoxiden**
Process for the preparation of aralkyl hydroperoxides
Procédé pour la préparation d'hydroperoxydes d'aralkyles

(30) Priorität: 09.11.1988 DE 3838028
(43) Veröffentlichungstag der Anmeldung: 16.05.1990
(73) Patentinhaber: Peroxid-Chemie GmbH, 82049 Pullach (DE)
(72) Erfinder: Dorn, Maximilian, Dr., D-8023 Pullach (DE); Hägel, Eberhard, Dr., D-8021 Icking (DE); Zeiss, Werner, Dr., D-8038 Gröbenzell (DE)
(74) Vertreter: Huber, Bernhard, Dipl.-Chem.

(56) Entgegenhaltungen:
- US-A- 4 267 387
- CHEMICAL ABSTRACTS, Band 79, 1973, Seite 431, Spalte 2, Zusammenfassung Nr. 5088a, Columbus, Ohio, US; A. BURGHARDT et al.: "Synthesis of cumene hydroperoxide from dimethylphenylcarbinol and hydrogen peroxide"
- CHEMICAL ABSTRACTS, Band 76, 1972, Seite 423, Zusammenfassung Nr. 153277q, Columbus, Ohio, US; M.S. BELEN'KII et al.: "Preparation of aliphatic-aromatic hydroperoxides by the oxidation of dimethylphenylcarbinol and alpha,alpha'-dihydroxy-diisopropylbenzenes"

## Beschreibung

Die Erfindung betrifft ein Verfahren zur Herstellung von Aralkylhydroperoxiden.

Hydroperoxide finden in der chemischen Industrie und Technik eine vielfältige Verwendung, z.B. als Katalysatoren oder Härter bei Polymerisationsreaktionen, als Sauerstofflieferanten in Treibmitteln, sowie als Zwischenprodukte für die Herstellung der entsprechenden Alkohole von Peroxiden und von Persäureestern. In dieser Hinsicht sind insbesondere die tert.-Aralkylhydroperoxide von großer Bedeutung.

Die tert.-Aralkylhydroperoxide werden im allgemeinen durch Oxidation der entsprechenden Kohlenwasserstoffe mit molekularem Sauerstoff hergestellt, wobei Konversionsraten von nur 30 bis 35 % erhalten werden; bei höheren Konversionsraten nimmt die Selektivität stark ab, d.h. es werden im vermehrten Maße Nebenprodukte gebildet. Die Hydroperoxide müssen dann durch Destillation, Kristallisation oder Extraktion aufkonzentriert oder vom nicht umgesetzten Ausgangskohlenwasserstoff abgetrennt werden.

Für die Herstellung von tert.-Hydroperoxiden durch Umsetzung von tert.-Alkoholen mit Wasserstoffperoxid unter Säurekatalyse sind aus der Literatur zwei Verfahren bekannt, die mit unterschiedlichen Konzentrationen und Mengen an Schwefelsäure arbeiten: R. Criegee und H. Dietrich (Annalen 560 (1948) 135) arbeiten unter Verwendung von 80 bis 95 %igem H₂O₂ und in Gegenwart von wenig konzentrierter Schwefelsäure; und Milas und Harries (J.Am.Chem.Soc. 60 (1938) 2434) arbeiten unter Verwendung von 30 %igem H₂O₂ in Gegenwart von viel 70 %-iger Schwefelsäure. Das Verfahren nach R. Criegee und H. Dietrich wird von den Autoren selbst als für größere Ansätze ungeeignet beschrieben, und angegeben, daß das ältere Verfahren nach Milas und Harries billiger und gefahrloser und daher für größere Ansätze allein brauchbar ist. Im Falle der Herstellung von tert.-Aralkylhydroperoxiden hat aber auch das Arbeiten nach Milas und Harries Nachteile: Diese Hydroperoxide sind sehr empfindlich gegen starke Säuren und zerfallen in Ketone und Phenole.

Nach M.S. Belenkiy et al (The Soviet Chem.Ind. 1 (1972) 16) werden die Aryl-alkyl-carbinole, die in Mischungen aus der Luftoxidation von Alkylaromaten enthalten sind, mit Wasserstoffperoxid zu den Hydroperoxiden oxidiert. Dabei sind hohe Umsätze und damit gute Ausbeuten nur zu erzielen bei Anwendung hoher Temperaturen und/oder relativ hoher Säurekonzentrationen. Die angegebenen optimalen Bedingungen stellen dabei einen Kompromiß zwischen Bildung und Zersetzung der Hydroperoxide dar.

A. Burghardt et al (Chemia Stosowana, Ser. A, Band 13, Nr. 4 (1969), 335-342) beschreiben die Umsetzung von Methyl-ethyl-phenylcarbinol mit Wasserstoffperoxid in Gegenwart von Schwefelsäure und einem Lösungsmittel zum sek.-Butylbenzol-hydroperoxid; durch die hohe Menge an unpolarem Lösungsmittel (vierfache Menge des Carbinols) wird das gebildete Hydroperoxid vor der Zersetzung durch Säure geschützt.

Nach A. Burghardt et al. (Zess.Nauk.Politech.Slask., Chem. No. 60 (1972) 3-20) wird Cumolhydroperoxid aus Dimethylphenylcarbinol und Wasserstoffperoxid in Gegenwart eines Lösungsmittels hergestellt. Als saurer Katalysator werden Schwefelsäure und/oder saure Kationenaustauscher verwendet. Auch nach diesem Verfahren wird nicht ohne Lösungsmittel gearbeitet und kein reines Hydroperoxid hergestellt. Bei der Herstellung eines festen Hydroperoxids wäre auch die Abtrennung des Ionenaustauscherharzes äußerst schwierig.

Auch in eigenen Versuchen der Anmelderin zur Herstellung der Hydroperoxide in reiner Form aus den reinen Carbinolen mit H₂O₂/Mineralsäure zeigte sich eine mangelhafte Umsetzung, und damit geringe Ausbeute und Reinheit des Produktes, oder aber Neigung zur Zersetzung, die zu einer starken Verfärbung des Produkts führt. Die säurekatalysierte Zersetzung der tert.-Aralkylhydroperoxide ist außerdem stark exotherm und stellt deshalb auch ein hohes Sicherheitsrisiko dar.

Aufgabe der vorliegenden Erfindung war es daher, ein Verfahren zur Herstellung von tert.-Aralkylhydroperoxiden bereitzustellen, mit dem die vorstehend beschriebenen Nachteile vermieden werden können und mit dem es möglich ist, die tert.-Aralkylhydroperoxide mit hoher Ausbeute und Reinheit auf einfache und gefahrlose Weise herzustellen. Diese Aufgabe wird mit dem erfindungsgemäßen Verfahren gelöst.

Gegenstand der Erfindung ist ein Verfahren zur Herstellung von Aralkylhydroperoxiden der Formel (I) 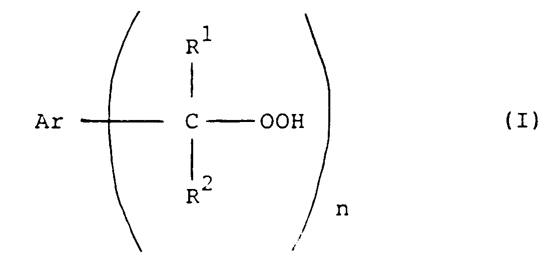
worin n = 1, 2 oder 3 ist,
Ar eine Phenylgruppe, die mit Halogen oder einer oder mehreren verzweigten oder geradkettigen Alkylgruppen mit 1 bis 5 C-Atomen substituiert sein kann oder eine Naphthylgruppe und jeder Rest R¹ und R² einen Niederalkylrest bedeutet, wobei die Alkylreste R¹ und R² zusammen 2 bis 4 Kohlenstoffatome besitzen, durch Umsetzung von Aralkylcarbinolen der Formel (II) 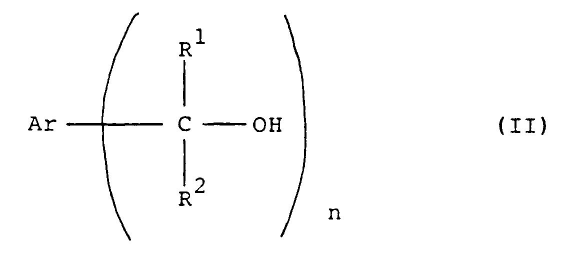
worin Ar, R¹, R² und n die für Formel (I) angegebene Bedeutung besitzen, mit Wasserstoffperoxid unter sauren Bedingungen, das dadurch gekennzeichnet ist, daß man die Umsetzung in Gegenwart eines säurebindenden Mittels durchführt.

Zweckmäßige Ausgestaltungen dieses Verfahrens sind Gegenstand der Ansprüche 2 bis 10.

Überraschenderweise hat es sich gezeigt, daß sich Aralkylhydroperoxide der Formel (I) durch Umsetzung von Aralkylcarbinolen der Formel (II) mit Wasserstoffperoxid unter sauren Bedingungen mit hohen Ausbeuten und sehr guter Reinheit herstellen lassen, wenn man die Umsetzung erfindungsgemäß in Gegenwart eines säurebindenden Mittels durchführt. Es treten dabei auch keine spontanen Zersetzungen auf, wodurch das Verfahren auch gefahrlos durchzuführen ist.

Als säurebindendes Mittel kann im allgemeinen jedes die Umsetzung nicht beeinflussende, also gegenüber den Reaktionspartnern inertes säurebindendes Mittel eingesetzt werden. Solche säurebindenden Mittel sind anorganische oder organische Verbindungen, die aufgrund ihres Dissoziationsgleichgewichts im sauren Reaktionsmedium als Protonenakzeptoren wirken können. Repräsentative Vertreter davon sind z.B. Salze anorganischer oder organischer Säuren mit Metallen, insbesondere Alkalimetallen oder Ammonium, oder mit organischen Kationen, wie z.B. Tetraalkylammoniumsalze. Unter diesen Verbindungen sind insbesondere zu nennen Phosphate, z.B. Alkali- oder Ammoniumphosphate, Borate, z.B. Borax, Carboxylate, z.B. Natriumacetat oder Ammoniumacetat, Harnstoffe usw.. Als besonders zweckmäßig und damit bevorzugt sind zu nennen Alkalisulfate, insbesondere Natriumsulfat, Ammoniumsulfat, Alkalihydrogensulfate, insbesondere Natriumhydrogensulfat und oder Ammoniumhydrogensulfat. Es können auch Gemische von zwei oder mehreren säurebindenden Mitteln verwendet werden, z.B. ein Gemisch aus Natriumsulfat und Natriumhydrogensulfat.

Vorzugsweise arbeitet man in Gegenwart von 1 bis 5 Mol Wasserstoffperoxid pro Mol Hydroxylgruppe des Carbinols und insbesondere mit 2 bis 3 Mol Wasserstoffperoxid pro Mol OH-Gruppe.

Die Wasserstoffperoxid-Konzentration in der wäßrigen Phase beträgt vorzugsweise 10 bis 35 Gew.-% und insbesondere 15 bis 25 Gew.-%. Als wäßrige Phase wird dabei das Gemisch (Lösung) aus Wasserstoffperoxid, Säure, dem säurebindenden Mittel (z.B. von Alkali- oder Ammoniumsulfat und/oder -hydrogensulfat) in Wasser verstanden.

Als Säure können die auf diesem Gebiet üblichen verwendet werden. Geeignet sind beispielsweise Phosphorsäure, Perchlorsäure und Sulfonsäure. Bevorzugt wird Schwefelsäure verwendet.

Vorzugsweise wird mit einer Säurekonzentration in der wäßrigen Phase von 1 bis 10 Gew.-% gearbeitet.

Die Temperatur der Umsetzung liegt vorzugsweise zwischen 15 und 70°C und insbesondere bei 35 bis 50°C.

Das zweckmäßigste Molverhältnis von Säure zu säurebindendem Mittel wird von der Art des umzusetzenden Ausgangscarbinols und den übrigen Reaktionsparametern (Temperatur, Menge und Konzentration an Wasserstoffperoxid und Säure) bestimmt und ist insbesondere abhängig von der Art des säurebindenden Mittels. In der Regel werden mit einem Molverhältnis Säure/säurebindendes Mittel = 1/2 bis 2/1 gute Ergebnisse erzielt. Bei Verwendung eines Gemisches aus zwei oder mehreren säurebindenden Mitteln, wie z.B. von Natriumsulfat und Natriumhydrogensulfat, kann auch das gegenseitige Verhältnis der einzelnen Komponenten des Gemisches eine Rolle spielen. So läßt sich z.B. unter Verwendung eines Gemisches aus Sulfat und Hydrogensulfat die Säurestärke der Mischung bei Veränderung des Verhältnisses von Sulfat/Hydrogensulfat variieren. Statt einer Mischung von Schwefelsäure und Alkali- oder Ammoniumsulfat kann auch eine Mischung von Schwefelsäure und eine diese teilweise neutralisierende Menge an Alkalihydroxid-oder Ammoniaklösung verwendet werden.

Die erfindungsgemäß hergestellten Aralkylhydroperoxide der allgemeinen Formel (I) sind in der Regel bei Raumtemperatur feste Stoffe. Deshalb kann es zur Einstellung einer geeigneten Konsistenz (Rührbarkeit) der Reaktionsmischung zweckmäßig sein, die Umsetzung in Gegenwart eines inerten Verdünnungsmittels (Phlegmatisierungsmittel) und/oder in Gegenwart eines inerten Lösungsmittels durchzuführen. Durch die Gegenwart eines für derartige Umsetzungen üblichen Phlegmatisierungsmittels kann auch die Explosionsneigung verringert werden.

Als inerte Lösungsmittel eignen sich z.B. hochsiedende aliphatische oder aromatische Kohlenwasserstoffe oder Phthalsäureester wie Dimethylphthalat oder Dibutylphthalat.

Die Menge an Verdünnungsmittel (Phlegmatisierungsmittel) und/oder an Lösungsmittel wird dabei vorzugsweise so gewählt, daß das Hydroperoxid noch in kristalliner Form und gut filtrierbar anfällt; das im festen Hydroperoxid verbleibende Lösungsmittel kann außerdem als Phlegmatisierungsmittel wirken und erhöht damit, wie auch die Zugabe eines zusätzlichen Phlegmatisierungsmittels, die Handhabungssicherheit des Peroxids. In der Regel ist es ausreichend, das Lösungsmittel in einer Menge von ca. 5 Gew.-%, bezogen auf das Ausgangscarbinol, zuzusetzen.

Die nachstehenden Beispiele sollen die Erfindung näher erläutern, ohne sie darauf zu beschränken. Wenn nicht anders angegeben, beziehen sich vorstehend und nachstehend die Temperaturangaben auf die Celsius-Skala und Mengenangaben auf Gewichtsteile und Gewichtsprozent.

### Beispiel 1:

In 1350 g 18 %igem Wasserstoffperoxid löst man 75 g Ammoniumsulfat. Nach Zugabe von 60 g Schwefelsäure (72 %ig) fügt man unter Rühren bei 25 °C portionsweise 245 g 1,4-Bis-(hydroxyisopropyl)benzol zu, heizt auf 50°C auf und rührt bei dieser Temperatur 2 Stunden. Nach Verdünnen mit Wasser kühlt man die Mischung auf 25°C ab, neutralisiert mit verdünnter Natronlauge, filtriert das fertige Produkt ab und wäscht es frei von Wasserstoffperoxid.

Man erhält 703 g eines weißen, wasserfeuchten Pulvers mit einem Gehalt von 35,2 % an p-Diisopropylbenzol-dihydroperoxid entsprechend 87,5 % der Theorie.

### Beispiel 2:

Zu einer Mischung von 1000 g 21 %igem Wasserstoffperoxid, 30 g 72 %iger Schwefelsäure und 30 g Ammoniumsulfat gibt man 15 g Dimethylphthalat und fügt dann unter Rühren bei 25°C portionsweise 245 g 1,4-Bis(hydroxyisopropyl)benzol zu, heizt auf 40°C auf und rührt bei dieser Temperatur 3 Stunden, wobei die Mischung dickflüssig wird. Nach Abkühlen auf 25°C wird die Reaktionsmischung mit verdünnter Natronlauge neutralisiert, das feste Produkt abfiltriert und von Wasserstoffperoxid freigewaschen.

Man erhält 640 g eines weißen, wasserfeuchten Pulvers mit einem Gehalt von 39,5 % an p-Diisopropylbenzol-dihydroperoxid, entsprechend 89,4 % der Theorie.

### Beispiel 3:

Zu einer Mischung von 1000 g 21 %igem Wasserstoffperoxid, 53 g Natriumhydrogensulfat und 15 g Dimethylphthalat gibt man unter Rühren bei 25°C portionsweise 245 g 1,4-Bis(hydroxyisopropyl)benzol zu, heizt auf 40°C auf und rührt bei dieser Temperatur 3 Stunden. Die dickflüssige Mischung wird mit Wasser verdünnt, auf 25°C abgekühlt und mit verdünnter Natronlauge neutralisiert. Das feste Produkte wird abfiltriert und von Wasserstoffperoxid freigewaschen.

Man erhält 677 g eines weißen, wasserfeuchten Produkts mit einem Gehalt von 35,7 % an p-Diisopropylbenzol-dihydroperoxid, entsprechend 85,4 % der Theorie.

### Beispiel 4 (Vergleichsbeispiel):

Man arbeitet wie im Beispiel 2 beschrieben mit dem Unterschied, daß kein Ammoniumsulfat zugegeben wird. Während der Nachrührzeit treten immer wieder Zersetzungserscheinungen auf, die sich durch Rauchentwicklung äußern. Besonders Spritzer auf der Gefäßwand zersetzen sich unter Rauchentwicklung und Braunverfärbung. Nach Abnutschen des Produkts ist die Mutterlauge gelbbraun verfärbt. Man erhält 659 g eines grauen, wasserfeuchten Pulvers mit einem Gehalt von 34 % an Dihydroperoxid, entsprechend 79,3 % der Theorie.

## Patentansprüche

1. Verfahren zur Herstellung von Aralkylhydroperoxiden der Formel (I) 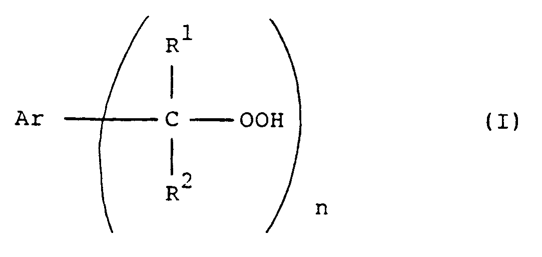 worin n = 1, 2 oder 3 ist,
Ar eine Phenylgruppe, die mit Halogen oder einer oder mehreren verzweigten oder geradkettigen Alkylgruppen mit 1 bis 5 C-Atomen substituiert sein kann oder eine Naphthylgruppe und jeder Rest R¹ und R² einen Niederalkylrest bedeutet, wobei die Alkylreste R¹ und R² zusammen 2 bis 4 Kohlenstoffatome besitzen, durch Umsetzung von Aralkyl-carbinolen der Formel (II) 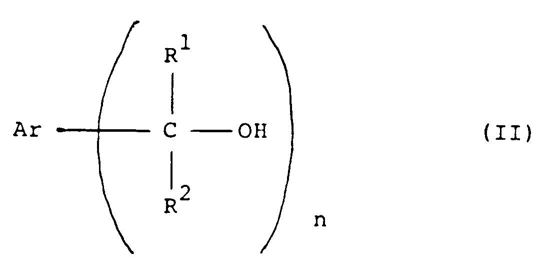 worin Ar, R¹, R² und n die für Formel (I) angegebene Bedeutung besitzen, mit Wasserstoffperoxid unter sauren Bedingungen,
**dadurch gekennzeichnet,**
daß man die Umsetzung in Gegenwart eines säurebindenden Mittels durchführt.

2. Verfahren nach Anspruch 1,
**dadurch gekennzeichnet,**
daß man als säurebindendes Mittel ein Alkalisulfat, Ammoniumsulfat, Alkalihydrogensulfat und/oder Ammoniumhydrogensulfat verwendet.

3. Verfahren nach Anspruch 1 oder 2,
**dadurch gekennzeichnet,**
daß man in Gegenwart von 1 bis 5 Mol, insbesondere von 2 bis 3 Mol Wasserstoffperoxid pro Mol OH-Gruppe arbeitet.

4. Verfahren nach einem der Ansprüche 1 bis 3,
**dadurch gekennzeichnet,**
daß die Wasserstoffperoxid-Konzentration in der wäßrigen Phase 10 bis 35 Gew.-% beträgt.

5. Verfahren nach Anspruch 4,
**dadurch gekennzeichnet,**
daß die Wasserstoffperoxid-Konzentration 15 bis 25 Gew.-% beträgt.

6. Verfahren nach einem der Ansprüche 1 bis 5,
**dadurch gekennzeichnet,**
daß die Säurekonzentration in der wäßrigen Phase 1 bis 10 Gew.-% beträgt.

7. Verfahren nach einem der vorhergehenden Ansprüche,
**dadurch gekennzeichnet,**
daß man als Säure Schwefelsäure verwendet.

8. Verfahren nach einem der Ansprüche 1 bis 7,
**dadurch gekennzeichnet,**
daß das Molverhältnis Säure/säurebindendes Mittel = 1/2 bis 2/1 beträgt.

9. Verfahren nach einem der Ansprüche 1 bis 7,
**dadurch gekennzeichnet,**
daß man bei einer Reaktionstemperatur von 15 bis 70°C, insbesondere von 35 bis 50°C arbeitet.

10. Verfahren nach einem der Ansprüche 1 bis 9,
**dadurch gekennzeichnet,**
daß man in Gegenwart eines inerten Verdünnungsmittels/Phlegmatisierungsmittels arbeitet.

11. Verfahren nach einem der Ansprüche 1 bis 10,
**dadurch gekennzeichnet,**
daß man in Gegenwart eines inerten Lösungsmittels arbeitet.

## Claims

1. Process for the production of aralkyl hydroperoxides of the formula (I) 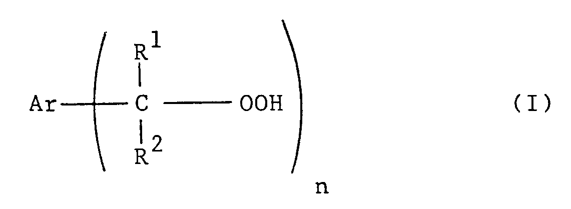 wherein n = 1, 2 or 3, Ar signifies a phenyl group, which can be substituted with halogen or one or more branched or straight-chained alkyl groups with 1 to 5 C-atoms, or a naphthyl group and each radical R¹ and R² signifies a lower alkyl radical, whereby the alkyl radicals R¹ and R² together possess 2 to 4 carbon atoms, by reaction of aralkylcarbinols of the formula (II) 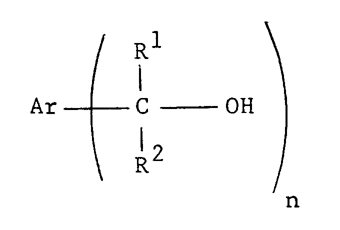 wherein Ar, R¹, R² and n possess the meanings given for formula (I), with hydrogen peroxide under acidic conditions, characterised in that one carries out the reaction in the presence of an acid-binding agent.

2. Process according to claim 1, characterised in that, as acid-binding agent, one uses an alkali metal sulphate, ammonium sulphate, alkali metal hydrogen sulphate and/or ammonium hydrogen sulphate.

3. Process according to claim 1 or 2, characterised in that one works in the presence of 1 to 5 mole, especially of 2 to 3 mole of hydrogen peroxide per mole of OH groups.

4. Process according to one of claims 1 to 3, characterised in that the hydrogen peroxide concentration in the aqueous phase amounts to 10 to 35 wt.%.

5. Process according to claim 4, characterised in that the hydrogen peroxide concentration amounts to 15 to 25 wt.%.

6. Process according to one of claims 1 to 5, characterised in that the acid concentration in the aqueous phase amounts to 1 to 10 wt.%.

7. Process according to one of the preceding claims, characterised in that one uses sulphuric acid as acid.

8. Process according to one of claims 1 to 7, characterised in that the mole ratio of acid/acid-binding agent amounts to = 1/2 to 2/1.

9. Process according to one of claims 1 to 7, characterised in that one works at a reaction temperature of 15 to 70°C, especially of 35 to 50°C.

10. Process according to one of claims 1 to 9, characterised in that one works in the presence of an inert dilution agent/desensitising agent.

11. Process according to one of claims 1 to 10, characterised in that one works in the presence of an inert solvent.

## Revendications

1. Procédé de préparation d'hydroperoxydes d'aralkyle de formule (I) 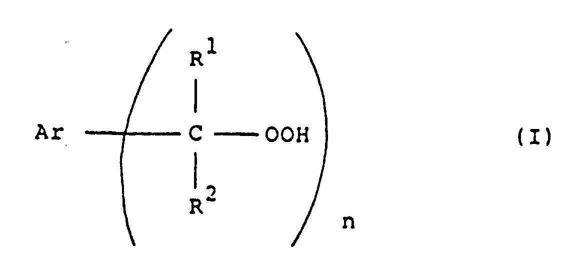 dans laquelle n=1,2 ou 3,
Ar représente un groupe phényle, qui peut être substitué par un halogène ou par un ou plusieurs groupes alkyle linéaire ou ramifié de 1 à 5 atomes de carbone, ou un reste naphtyle, et chacun des restes R¹ et R² représente un reste alkyle inférieur, les restes alkyle R¹ et R² possédant ensemble 2 à 4 atomes de carbone, par réaction d'aralkylcarbinols de formule (II) 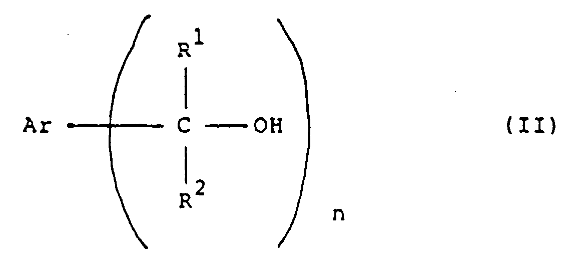 dans laquelle Ar, R¹, R² et n ont la signification indiquée pour la formule (I), avec du peroxyde d'hydrogène dans des conditions acides,
caractérisé en ce que l'on effectue la réaction en présence d'un agent fixateur d'acide.

2. Procédé selon la revendication 1, caractérisé en ce que l'on utilise comme agent fixateur d'acide un sulfate alcalin, du sulfate d'ammonium, un hydrogénosulfate alcalin et/ou de l'hydrogénosulfate d'ammonium.

3. Procédé selon la revendication 1 ou 2, caractérisé en ce que l'on travaille en présence de 1 à 5 moles, en particulier de 2 à 3 moles, de peroxyde d'hydrogène par mole de groupe OH.

4. Procédé selon l'une des revendications 1 à 3, caractérisé en ce que la concentration de peroxyde d'hydrogène dans la phase aqueuse est de 10 à 35 % en masse.

5. Procédé selon la revendication 4, caractérisé en ce que la concentration de peroxyde d'hydrogène est de 15 à 25 % en masse.

6. Procédé selon l'une des revendications 1 à 5, caractérisé en ce que la concentration d'acide dans la phase aqueuse est de 1 à 10 % en masse.

7. Procédé selon l'une des revendications précédentes, caractérisé en ce que l'on utilise comme acide de l'acide sulfurique.

8. Procédé selon l'une des revendications 1 à 7, caractérisé en ce que le rapport molaire acide/agent fixateur d'acide est de 1/2 à 2/1.

9. Procédé selon l'une des revendications 1 à 7, caractérisé en ce que l'on travaille à une température de réaction de 15 à 70°C, en particulier de 35 à 50°C.

10. Procédé selon l'une des revendications 1 à 9, caractérisé en ce que l'on travaille en présence d'un diluant/stabilisant inerte.

11. Procédé selon l'une des revendications 1 à 10, caractérisé en ce que l'on travaille en présence d'un solvant inerte.
